# EUROPÄISCHE PATENTANMELDUNG

(11) **EP 1 745 795 A2**
(43) Veröffentlichungstag der Anmeldung: **24.01.2007**
(21) Anmeldenummer: 06117493.4
(22) Anmeldetag: 19.07.2006
(51) Int. Cl.: A61K 36/00, A61K 35/56, A61P 25/28

(54) **Pharmzeutische Wirkstoff-Kombination zur Behandlung von Multipler Sklerose**

(30) Priorität: 19.07.2005 DE 102005033645
(71) Anmelder: TOXIMED GmbH, 80331 München (DE)
(72) Erfinder: Weickmann, Dirk, 80339, München (DE)
(74) Vertreter: Behnisch, Werner

(57) **Zusammenfassung**

Pharmazeutische Wirkstoff - Kombination zur Behandlung von Multipler Sklerose, bestehend aus Wirkstoffen die gewonnen werden aus der Rinde von speziellen Weidenarten, dem Mundsekret bestimmter Geckoarten und dem Mundsekret bestimmter Eidechsenarten.

## Beschreibung

Seit Menschengedenken nutzen wir Kräuter, Wurzeln, Baumrinde und Pflanzensäfte als Arzneistoffe. Das Wissen um ihre Wirkung wurde von Generation zu Generation weitergegeben. Heute helfen uns die Vorbilder aus der Natur, um mit modernen Methoden zu neuen Wirkstoffen zu gelangen.
Viele der heute angewandten Arzneimittel beruhen auf altbekannten Quellen. Das Herzmedikament Digitalis stammt beispielsweise aus dem Fingerhut, einer seit dem Altertum bekannten giftigen Blütenpflanze. Der Wirkstoff im Aspirin leitet sich von Inhaltsstoffen der Weidenrinde ab. Und einige Betäubungsmittel haben ihren Ursprung im Morphin aus dem Schlafmohn. Jede dritte Pflanze, aus der wir Medikamente herstellen, kommt aus dem Regenwald. Dabei ist gerade einmal ca. 1 Prozent der dort vorkommenden Pflanzenarten erforscht. Mit dem Aussterben von Pflanzenarten drohen mögliche Kandidaten für Arzneien der Zukunft für immer zu verschwinden. Denn auch die moderne Pharmakologie nutzt natürliche Wirkstoffe als Ausgangssubstanzen, um durch chemische Veränderungen die Eigenschaften eines Arzneistoffs nach Wunsch zu beeinflussen.
Viele Pflanzen und Tiere verwenden zum Schutz ihres eigenen Lebens und zum eigenen Nahrungserwerb, auf ihren speziellen Organismus und seine besonderen Bedürfnisse abgestimmte, so genannte biogene, Gifte. Diese biogenen Gifte haben im Laufe langer Entwicklungszeiträume ihren Platz gefunden im Zusammenspiel der verschiedenen Arten von Leben.
Deshalb erkennt auch heute noch jedes erwachsene Wildtier gefährliche Pflanzen und giftige Tiere seiner natürlichen Umgebung.
Dabei können Pflanzen oder Tiere durch die Produktion von Giftstoffen primär giftig wirken oder erst durch die Aufnahme toxischer Substanzen aus der belebten oder unbelebten Umwelt sekundäre Toxizität erhalten.
Die Nutzung dieser biogenen Gifte begann in der Geschichte der Menschheit schon in der Urzeit als sie zur Erlegung von Beutetieren mit vergifteten Waffen diente.
Zur gefahrlosen Anwendung dieser Gifte waren jedoch von Anfang an gewisse Grundkenntnisse über deren Behandlung und Wirksamkeit erforderlich.

Die weiter durchgeführten Versuche, die Zusammensetzung des chemischen Aufbaus biogener Gifte zu entschlüsseln, führten später zur gezielten Suche bestimmter Wirkstoffe als eigentliche Verursacher beobachteter Wirkungen.

Insbesondere nach der von Paracelsus (1493 - 1541) erhobenen Forderung, die Wirkstoffe von Arzneipflanzen zu isolieren, die zur Entwicklung der latrochemie, also der Chemie hinsichtlich ihres ärztlichen Anwendungsbereichs, beitrug, dürften diese Bemühungen verstärkt haben. Vor allem die Kunst des Destillierens von Stoffen wurde in den Dienst der Forschung gestellt und lieferte eine Vielzahl ätherischer Öle und flüchtiger Stoffe. Aber für die Isolierung anderer Wirkstoffe oder gar für deren chemische Aufschlüsselung waren die damals bekannten Methoden unzureichend. Erst zu Beginn des 19. Jahrhunderts war die Entwicklung der technischen Fertigkeiten in der Chemie weit genug fortgeschritten, die Ära der Isolierung von reinen Wirkstoffen aus biologischem Material einzuleiten.
Zunächst nutzte man, zur Abtrennung der gesuchten Wirkstoffe von den Begleitstoffen, die Unterschiede in der Löslichkeit der untersuchten Substanzen in verschiedenen Lösungsmitteln. Beobachtet wurden hierbei, zum Beispiel mit Fällungsmitteln, die Unterschiede im Verteilungsverhalten zwischen zwei nicht mischbaren flüssigen Phasen, in der Flüchtigkeit und in der chemischen Reaktivität,

Einen gewaltigen Aufschwung in der Trenntechnik, dem Weg zur Ermittlung von Wirkstoffen zur Bekämpfung von Krankheiten, machte die Entwicklung chromatographischer Verfahren in der Mitte des 20. Jahrhunderts möglich. Ausgehend von der Verteilung zwischen einer mobilen und einer stationären flüssigen Phase, von der Adsorption, den Molekülsiebeffekten, dem lonenaustausch, der Affinität (insbesondere von Proteinen) zu bestimmten chemischen Verbindungen (z.B. Enzymsubstraten) und der Beweglichkeit geladener Moleküle im elektrischen Feld, wurde eine Vielzahl neuer Trenntechniken entwickelt

Derzeit werden Tumore, als die gefährlichsten und gefürchtetsten Krankheiten unserer Zeit auf eine sehr radikale und wenig umweltschonende Weise bekämpft. Als einfache kennzeichnende Schlagworte können hier gelten:
Stahl, Strahl und Chemotherapie.
Das bedeutet einmal, dass Tumore, falls einigermaßen erreichbar, im Prinzip mit dem Stahl eines Messers herausgeschnitten, durch eine breit gefächerte Bestrahlung verbrannt, oder über eine so genannte Chemotherapie mit , auch gesunde Zellen angreifenden, aggressiven Zytostatika zerstört werden.
Sowohl bei normalen Behandlungen mit dem Skalpell als auch mit ionisierender Strahlung ist eine räumliche Begrenzung des Operationsgebiets nicht möglich. Es werden zwangsläufig auch gesunde Körperzellen vernichtet. Die unerwünschten Nebenwirkungen der Chemotherapie sind allgemein bekannt.
Im Gegensatz hierzu wurde aber auch versucht eine Krebstherapie die ihren Namen verdient auf subtilere Weise zu ermöglichen. Zu diesem Zweck wurde auf den reichen Schatz der Natur zurückgegriffen. Es werden hierzu, unter anderen, viele aus giftigen Lebewesen isolierte, stark wirksame Stoffe in therapeutischen Dosen als Arzneistoffe genutzt
So ist aus der DE 199 61 141 A1 ein pharmazeutischer Wirkstoff bekannt, bei dem gefunden wurde, dass Bestandteile der Spinnengifte von Spinnen der Familie Sicaridae zur Behandlung von Tumorerkrankungen verwendet werden können.
Es werden hierbei in der Hauptsache ein Peptidtoxin aus dem Gift dieser Spinnenart, eine weitere aus dem Gift gewonnene antagonistisch wirkende Substanz und / oder eine Kombination dieser Bestandteile medizinisch genutzt.
Es kann dieser Wirkstoff zur Behandlung von Tumorerkrankungen sowie parallel bzw. unterstützend zu Tumoroperationen eingesetzt werden und Rest ― Tumorgewebe zerstört werden. Bei der Therapie können genetisch veränderte Körperzellen (Tumorzellen) zerstört werden, da der betreffende Wirkstoff die veränderte Oberflächenstruktur solcher Zellen erkennt und komplikationsfrei abtötet.. Der Gesamtgiftgehalt dieser Spinnenart, sozusagen ein Cocktail verschiedener Substanzen, ist auf Grund seiner bereits in geringen Dosen letalen Wirkung, nicht pharmazeutisch einsetzbar.
Es wurden jedoch auch schon andere biogene Substanzen, wie zum Beispiel Mundsekret, als Grundlage pharmazeutischer Wirkstoffe erprobt.

Die Anwendung von biogenen Giften, bzw.- Substanzen als Grundlage von pharmazeutischen Wirkstoffen gegen Tumore ließ bisher die Behandlung anderer Krankheiten wie zum Beispiel von Multipler Sklerose (MS) Acht.

Im Folgenden wird die Multiple Sklerose kurz beschrieben.

Vermutlich müssen mehrere Bedingungen und Einflüsse zusammentreffen, damit die Multiple Sklerose ausgelöst wird. Das Durchschnittsalter bei Beginn der Erkrankung liegt im frühen Erwachsenenalter. Frauen erkranken daran öfter als Männer, wobei sich hierfür noch keine Erklärung fand. Die MS ist die häufigste entzündliche neurologische Erkrankung in Deutschland.
Es gibt Untersuchungen, die aufzeigen, dass in bestimmten Breitengraden der Erde MS häufiger auftritt, in anderen dagegen selten. So ist die MS in Mitteleuropa, Nordeuropa und Nordamerika häufiger, als in Südeuropa, Südamerika und Afrika. Dabei sollte man aber nicht übersehen, dass es in den südlichen Ländern schlechtere Diagnosemöglichkeiten gibt. Eine passende Theorie dazu ist, dass das Ausmaß der Sonneneinstrahlung einen Einfluss auf die Entwicklung von MS hat.
Zur Erkrankung gibt es wegen der vielen unterschiedlichen Verläufe von MS auch verschiedene Ursachen - Theorien. Zu nennen ist hier die Autoimmuntheorie, die Virus - Hypothese, die genetischen Faktoren, die Stoffwechseltheorie und die psychosomatischen Theorien.
Die Forschung von Wissenschaftlern und Ärzten kommt nur mühsam voran, zum einen, weil man das zentrale Nervensystem am lebenden Menschen nicht operativ öffnen kann und zum zweiten, weil die notwendigen finanziellen Mittel fehlen.
Des Weiteren wird kolportiert, dass Innovationen auf diesem Gebiet, ebenso wie bei AIDS, die Interessenlage großer Konzerne tangieren.

Die Multiple Sklerose kann in vielen Erscheinungsformen auftreten.
Bei ca. 40% aller MS - Erkrankungen kommt es zur Entzündung eines Sehnervs, Bemerkbar macht sich die Sehnerventzündung durch einen Verlust an Sehschärfe und das Sehen verschwommener Bilder. Es können Schmerzen bei Augenbewegungen auftreten. Es kann auch zu Lähmungen der Augenmuskeln kommen. Dabei sehen die Betroffenen meist Doppelbilder. Augenzittern kann auftreten, das jedoch nur bei starker Ausprägung zu Sehstörungen führt. Diese Beschwerden können völlig verschwinden, aber auch immer wieder auftreten. In manchen Fällen besteht ein Fremdkörpergefühl im Auge.

Im Hirnstamm, der Verbindung zwischen Gehirn und Rückenmark, verlaufen, unter anderem, Nervenfasern für die Berührungsempfindlichkeit des Gesichts. Werden diese von MS befallen, kann es zu heftigen Schmerzattacken im Gesicht kommen besonders bei Berührung. Hier reicht schon der Kontakt der Haut mit längeren Haaren.
Schwindel und Brechreiz können vorkommen wenn die Nervenbahnen geschädigt werden, die eine Verbindung zum Gleichgewichtsorgan herstellen. Entzündungsherde im Hirnstamm können weiterhin zu einer verwaschenen Sprache führen. Es kann auch zu Sprachstörungen kommen, die nur für kurze Zeit, oft mehrmals am Tag, auftreten.

Werden Nervenfasern im Bereich des Kleinhirns oder in seinen Verbindungsbahnen von der MS befallen, treten Koordinationsstörungen auf. Sichtbar ist dies zum Beispiel durch Zittern, durch einen unsicheren Gang und durch eine abgehackte oder Iallende Sprache. Je nach Ausprägungsgrad sind Sprachstörungen besonders behindernd und können den Erkrankten sozial isolieren. Der Erkrankte wird damit des wichtigsten Kommunikationsmittels mit seiner menschlichen Umwelt beraubt. Es erfordert hierbei sowohl vom MS - Kraken als auch vom Gesprächspartner viel Geduld, immer wieder zu versuchen, das Gespräch langsam und ruhig zu führen.

Es bestehen Schwierigkeiten, gezielte Bewegungen auszuführen, wie zum Beispiel Getränke an den Mund zu führen. Das Zittern kann so stark sein, dass geplante Bewegungen nicht ausgeführt werden können. Das führt dann oft zu Wutausbrüchen, wobei der Betroffene sich selbst die Schuld an den Unvermögen gibt. Oft wird dieses Unvermögen durch erneute Versuche verstärkt, weil es einfach nicht akzeptiert werden kann, dass der Körper bestimmt, was vollführt werden kann und was nicht. Es können Unsicherheiten beim Sitzen, Stehen oder Gehen auftreten. Der unsichere Gang bei einem an MS Erkrankten wird von Außenstehenden oft mit dem Gang eines Betrunkenen verwechselt. Dies führt zwangsläufig zu peinlichen und kränkenden Situationen. Viele MS - Betroffene fühlen sich beim Gehen behindert, gerade dann wenn sie sich besonders darauf konzentrieren.

Bei Befall des Rückenmarks mit Multipler Sklerose können grundsätzlich zwei verschiedene Störungen auftreten. Auf der einen Seite kann es zu Störungen der Empfindungswahrnehmungen kommen, auf der anderen Seite zu Störungen der Bewegung der Muskeln.
Bei einer Entzündung des Rückenmarks kann es zu Sensibilitätsstörungen kommen in der Form von Kribbeln und Taubheitsgefühlen in der Muskulatur. Diese können dann als Schwere, als Kälte, Beengung oder Spannung empfunden werden. In den Händen kann es zu einer Empfindung kommen als sei in und zwischen den Fingern Pelz, oder als seien die Hände mit einem Pelz überzogen, Es kann auch zu einer schmerzhaften Missempfindung kommen, die meist als Brennen in Erscheinung tritt. Solche Missempfindungen sind besonders störend, da bei jeder Berührung mit den Händen ein unangenehmes Gefühl entsteht.
Nervenstörungen der Muskeln führen zu unterschiedlichen Beschwerden. Die Muskeln können schwach und schlaff werden. So können die Beine wegknicken oder ein Fuß hängt schlaff nach unten. Aber auch eine beständige Anspannung (Spastik) ist möglich, so dass bestimmte Bewegungen nicht mehr oder nur noch unter Anstrengung machbar sind .Dazu gehört u.a. die Streckspastik oder Beugespastik im Bein. In diesem Fall bekommt man das Bein nicht mehr gestreckt oder gebeugt wenn man aufstehen will.
Da die motorischen Störungen meist das Gehvermögen beeinflussen, beeinträchtigen sie oft auch die allgemeine Leistungsfähigkeit.
Weitere Beschwerden bei Befall des Rückenmarks von MS sind Blasenstörungen und Darmstörungen. Die Angst vieler MS - Kranker, bei einem Harndrang nicht rechtzeitig eine Toilette besuchen zu können, führt oft dazu, dass sie nicht mehr aus dem Haus gehen möchten.
Harnträufeln und Harndrang oder der vollständige Verlust über die Kontrolle der Harnblase oder des Darms sind die Beschwerden, die zu den größten sozialen Beeinträchtigungen führen können. Auch der Harn - oder Blasenverhalt ist unangenehm. Besonders der Harnverhalt, bei dem die Blase nicht vollständig entleert werden kann, kann zu schmerzhaften Blaseninfektionen führen.

Da es Anzeichen dafür gab dass es sich bei MS um eine Krankheit handelt bei der als Erreger so genannte "langsame Viren" oder "Slow ― Viruses" eine maßgebliche Rolle spielen, wurde bei der Entwicklung eines Wirkstoffes dieser Gesichtspunkt berücksichtigt.

Es ist die Aufgabe des erfindungsgemäßen Wirkstoffs die beschriebenen Beschwerden beim Auftreten von durch Slow - Viruses verursachten Krankheiten, wie Multipler Sklerose, zu beheben oder zumindest auf ein erträgliches Maß zu reduzieren

Diese Aufgabe wird gelöst von einem Wirkstoff mit der Merkmalskombination des Anspruchs 1, sowie dem Verfahren zur Herstellung nach Anspruch 9.

Es wird bei dem erfindungsgemäßen Wirkstoff nicht nur generell von dem Ansatz ausgegangen, biogene Grundbestandteile zu verwenden, sondern es wird ein Wirkstoff offenbart, der zur Erhöhung seiner Wirksamkeit aus einer Kombination von drei, auf verschiedene Weise gewonnenen, biogenen Bestandteilen beruht.

### 1.Bestandteil des erfindungsgemäßen Wirkstoffs

Der 1. Bestandteil des erfindungsgemäßen Wirkstoffs wird aus dem Mundsekret von Tieren der Gattung Rhacodactylus, insbesondere von einer der folgenden Echsen gewonnen:
a) Rhacodactylus auriculatus (Höckerkopfgecko)
b) Rhacodactylus leachianus (Riesengecko)
c) Rhacodactylus leachianus henkeli (Henkels Riesengecko)
d) Rhacodactylus chahoua (Flechtengecko)
e) Rhacodactylus ciliatus (Kronengecko)

Die Familie der Geckos (Geckonidae) bevölkert seit etwa 50 Millionen Jahren die Erde und hat sich im Laufe ihrer Entwicklung über die ganze Erde verbreitet. Dank ihrer hervorragenden Anpassungsfähigkeit haben die Geckos die verschiedensten Lebensräume erobert und sind sowohl in den gemäßigten Zonen wie auch in den Wüsten der Erde und den Tropen anzutreffen. Dort haben sie es zu eine schier unüberschaubaren Artenvielfalt gebracht.
Bis heute sind ca. 90 Gattungen mit 960 Arten bekannt, die abhängig von Gestalt und Abstammung in vier Unterfamilien unterteilt werden (H. Rösler: Geckos der Welt)
Eublepharinae (Lidgeckos)
Diplodactyliniae (Doppelfingergeckos)
Gekkoninae (Eigentliche Geckos)
Spaerodactylinae (Kugelfingergeckos)

Dabei sind Geckos kleine bis mittelgroße Echsen, die zwischen 4 cm und knapp 40 cm groß werden können. Die meisten Geckos (ca. 75%) sind dämmerungs- oder nachtaktive Tiere mit dementsprechend unauffälliger Färbung und einer der Nachtaktivität angepassten Spaltpupille. Manchmal sonnen sich diese scheuen und meist sehr flinken Tiere aber auch in der frühen Dämmerung oder am Tage so wie es ihre Verwandten, die so genannten Taggeckos gerne tun.
Diese sind auffälliger gefärbt und besitzen eine runde Pupille.

Alle Rhacodactylus Arten sind auf den neukaledonischen Inseln und Australien beheimatet.
Der Höckerkopfgecko wird auch als gargoylegecko bezeichnet, was sich auch als Koboldgecko übersetzen lässt. Im Gegensatz zu anderen Arten neigt der Höckerkopfgecko dazu, große Mengen Obst zu essen. Er bevorzugt dabei Aprikosen, Bananen und Pflaumen. Er stellt die kleinste Art dar. und wird etwa 12 bis 13 Zentimeter groß.

Die neukaledonischen Riesengeckos zählen zu den größten Geckos der Welt. Die Gattung ist mit sechs Arten auf Neukaledonien und den umliegenden Inseln beheimatet. Die Art ist durch den Verlust an Lebensraum potentiell gefährdet. Trotz ihres gedrungenen Körpers und einer Körperlänge von über 35 Zentimetern verschmelzen die Tiere praktisch visuell mit der Umgebung. Gründe hierfür sind die Färbung, die Oberflächenstruktur der Haut und die gefransten Hautsäume, Diese Tarntracht dient der Feindvermeidung und erleichtert diesem Lauerjäger den Beutefang.
Ein wichtiger Orientierungssinn dieser Tierart ist der Geruchssinn. Dieser ermöglicht es dem nachtaktiven Gecko, auch im Dunkeln reife Früchte oder Geschlechtspartner zielgenau lokalisieren zu können. Das Sehvermögen dient wohl vor allem der Feinderkennung. Die Augen besitzen keine Lider sondern nur ein durchsichtiges Häutchen, die so genannte Brille. Mit der Zunge werden die Augen gereinigt und befeuchtet. Die senkrecht geschlitzte Pupille ist äußerst flexibel und erlaubt eine Orientierung unter unterschiedlichen Lichtbedingungen. Diese Tiere verfügen auch über einen guten Gehörsinn, spielen doch Lautäusserungen bei der innerartlichen Kommunikation eine wichtige Rolle.

Eine spezielle Art Rhacodactylus leachianus henkeli (Henkels Riesengecko) ist nach ihrem Entdecker Friedrich Wilhelm Henkel benannt,

Eine weitere Art ist der Rhacodactylus chahoua oder auch Flechtengecko.
Er hat eine Körperlänge von ca. 15 Zentimetern, wobei der Schwanz noch einmal dieselbe Länge erreicht. Seine Färbung ist in hohem Maß variabel.

Rhacodactylus ciliatus (Guichenot 1866) oder auch Kronengecko erreicht bis 21 cm Gesamtlänge. Die Färbung reicht von grau, braun, grün und blassgelb bis rostrot. Die Iris ist goldbraun. Am Schläfenrand zieht sich eine Reihe vergrößerter Stachelschuppen entlang, was dem Kronengecko zu seinem Namen verhalf. Er ist nachtaktiv und frisst Früchte und Insekten. Der Kronengecko verfügt über eine beachtliche Sprungkraft.
Er ist verbreitet im Süden Neukaledoniens und auf der Ile des Pins.

Der 1.Bestandteil des erfindungsgemäßen Wirkstoffs wird dem Mundsekret der unter a) bis e) angeführten Arten Rhacodactylus entnommen.

Alle Vertreter dieser Familie sind omnivor, das heißt allesfressend.
Das bedeutet, dass diese Tiere neben tierischer Kost mehr oder weniger auch Früchte verzehren.
Da die Oberfläche der Früchte meist kontaminiert ist mit Bakterien und Protozoen haben die Geckos zum Schutz vor Krankheiten im Mundsekret Abwehrstoffe gegen Bakterien und Viren entwickelt.

Es wurde gefunden, dass diese Abwehrstoffe auch gegen so genannte "Slow - Virus - Infektionen" wirksam sind. Hierbei handelt es sich um entweder durch "konventionelle Viren" oder durch "unkonventionelle Agenzien" hervorgerufene übertragbare Erkrankungen des Zentralnervensystems mit bis zu Jahren dauernder Inkubationszeit und chronisch fortschreitenden, meist tödlichen, Funktionsstörungen.

Deshalb werden erfindungsgemäß die Eigenschaften der unter a) bis d) genannten Gecko - Arten dazu benutzt Multiple Sklerose -Erreger zu bekämpfen.

Die Gewinnung des erfindungsgemäßen Wirkstoffes erfolgt dadurch, dass von dem jeweiligen Gecko ein Wattestäbchen, beispielsweise Transwaps, abgeschleckt wird und dieses dann in isotonischer NaCl ― Lösung, einer Mischung, enthaltend eine isotonische NaCl-Lösung und Tarantula D4, oder sterilem Wasser ausgeschwenkt wird. Die Mischung, die isotonische NaCl-Lösung und Tarantula D4 enthält, kann daneben bevorzugt Ameisensäure und/oder einen Gesamtextrakt von Giften der Ameisen Buldog-ants enthalten. Insbesondere bevorzugt als Mischung ist eine Lösung aus 15 ml isotonischer NaCl-Lösung und 7 ml Tarantula D4, der wahlweise noch Ameisensäure und/oder ein Gesamtextrakt von Giften der Ameisen Buldog-ants beigefügt werden kann. Außerdem kann das derart hergestellt Lösungsmittel zusätzlich noch einen Giftcocktail der Gattung Loxosceles, wobei die Arten Loxosceles laeta, Loxosceles gaucho, Loxosceles Mallorca und Loxosceles Menorca bevorzugt sind, oder ein Mundraumsekret der Krustenechse Heloderma sp. und/oder Timon sp. enthalten.
Als Basisbestandteil des oben genannten Lösungsmittels werden in 15 ml 0,9 prozentiger NaCl ― Lösung 7ml der homöopathischen Substanz Tarantula D4 eingearbeitet. Tarantula D4 wird vermischt auf homöopathische Weise unter Schütteln zum Erdmittelpunkt mit 15 ml der 0,9 prozentigen NaCl ― Lösung. Zu der erhaltenen Lösung gibt man bis zu 0,5 ml einer gesättigten Lösung des gesamten Giftcocktails von Spinnen der Arten Loxosceles laeta oder Loxosceles gaucho oder Loxosceles Mallorca oder Loxosceles Menorca. Aufgrund unterschiedlicher Mengenverhältnisse der verwendeten Gesamtgift - Cocktails ist es teilweise erforderlich , den Gesamtgift - Cocktail in wasserfreier Ameisensäure anzureiben, der wiederum 1 bis 2 ml des Gesamtextrakts von Bulldog - ants zugemengt wird. Diese Menge bezieht sich auf eine Menge des Gesamtgift - Cocktails von 10 ml. Anstelle des Giftcocktails von Spinnen kann auch 1 ml eines Mundraumsekrets der Krustenechsen Heloderma sp. und / oder Timon sp vorsichtig, eventuell unter Erwärmung auf 30 °C , eingearbeitet werden. Beispiele möglicher Lösungsmittel sind in der DE 103 57 970.2 offenbart, deren Inhalt hiermit vollständig aufgenommen wird.
Das Abschlecken der Wattestäbchen ist leicht zu bewerkstelligen, da die Geckos die Angewohnheit haben die jeweiligen Früchte vor dem Verzehr abzuschlecken.
Durch Autoklavieren des Sekrets kann der erfindungsgemäße Wirkstoff haltbar gemacht werden, wobei die Wirkung erhalten bleibt.
Bei der Anwendung des erfindungsgemäßen Wirkstoffes in subkutaner Form oder intravenös konnte keine toxische Wirkung auf den Menschen (bzw. auf Primaten) beobachtet werden.
Für die Verwendung des erfindungsgemäßen Wirkstoffs bei der beginnenden Entwicklung von MS, bzw. AIDS, kann auch eine Kombination mit einem in einer früheren Patentanmeldung beschriebenen Wirkstoff gebraucht werden.
Wahlweise kann der erfindungsgemäße Wirkstoff eine zu dem jeweiligen Mundsekret antagonistische bzw. synergistische und / oder Durchdringungssubstanz aus dem. Mundsekret, der betreffenden Tierart enthalten.
Die antagonistisch bzw. synergistisch wirkende Substanz ist bevorzugt eine Phospholipase oder eine Hyaluronidase oder eine Kombination beider Substanzen Weiterhin ist möglich, dass die antagonistisch bzw. synergistisch wirkende Substanz eine Mischung aus den, in anderen Arten, vorhandenen Phospholipasen und Hyaluronidasen und / oder Toxinen ist.
Es kann das Mundsekret und die hierzu antagonistisch und / oder synergistisch wirksame Substanz durch ein Fraktionierungsverfahren aus dem Gesamt- Cocktail des Mundraumsekrets erhalten werden, und es ist weiterhin möglich, dass der pharmazeutische Wirkstoff ein Mundsekret und eine hierzu antagonistisch oder synergistisch wirkende Substanz enthält, die aus verschiedenen Fraktionen stammen. Dadurch kann der pharmazeutische Wirkstoff in seiner Wirkung vorteilhafterweise auf die zu behandelnde MS - Art abgestimmt werden.
Das Mundsekret und die hierzu antagonistisch und / oder synergistisch wirkende Substanz können durch an sich bekannte Fraktionierungsverfahren zur Auftrennung von Proteinen aus dem Mundssekret, erhalten werden. Es ist möglich, dass die gewonnen Substanzen und die hierzu antagonistisch oder synergistisch wirkende Substanz durch Gelchromatographie, HPLC, Affinitätschromatographie und / oder lonenaustauschchromatographie erhalten werden.

Bevorzugt ist außerdem, dass das Mundsekret in einer solchen Menge als pharmazeutischer Wirkstoff vorliegt, dass eine bezüglich kranken Zellen heilende Wirkung des Wirkstoffs erreicht wird.
Weiterhin werden die benötigten Mengenverhältnisse so gewählt, dass der erfindungsgemäße Wirkstoff keine oder nur eine geringe toxische Wirkung im zu behandelnden Patienten entfaltet. Selbstverständlich sind hierbei die Mengen der pharmazeutischen Wirkstoffe auch auf die Art der zu behandelnden Krankheit und die physischen, gegebenenfalls auch psychischen, Gegebenheiten des jeweiligen Patienten abzustimmen. Die für eine solche Abstimmung benötigten Vorversuche sind vom Fachmann im Rahmen von Tierversuchen und / oder ethisch vertretbaren Versuchen am Patienten aufgrund seines fachlichen Wissens und Könnens vorzunehmen.
Weiterhin bevorzugt ist ein pharmazeutischer Wirkstoff, bei dem der Menge an. Mundsekret, und der hierzu antagonistisch oder synergistisch wirkenden Substanz eine weitere Menge an homöopathischer Substanz, Enzymen und antagonistisch oder synergistisch wirkender Substanz beigefügt ist, die in Abhängigkeit von der zu behandelnden Krankheit gewählt wird.
Es ist weiter bevorzugt, dass der erfindungsgemäße pharmazeutische Wirkstoff übliche Träger - und Hilfsstoffe enthält, wie Antibiotika, Antimykotika, Antituberkulotika, Mittel gegen Parasiten, Zytostatika, Aminosäuren, die Wundheilung begünstigende Enzyme und / oder Mitosehemmstoffe. Bevorzugt sind hierbei Penicillin/Streptomycin, Polymyxin / Gentalmycin (5%), Mitopodozid, Vinca rosea - Alkaloide, Bromelaina oder Bromelains.
In diesem erfindungsgemäßen pharmazeutischen Wirkstoff werden das Mundsekret, und die antagonistisch oder synergistisch wirkende Substanz in Kombination mit der homöopathischen Substanz miteinander eingesetzt. Es ist aber auch möglich, die Einzelsubstanzen in pharmazeutischen Wirkstoffen zu benutzen und sich hierbei die speziellen Wirkungen der Einzelsubstanzen für eine therapeutische Anwendung nutzbar zu machen.
Es ist auch möglich die beschriebenen Wirkstoffe chemisch - synthetisch oder durch gentechnologische Methoden in rekombinierter Form herzustellen. Wie bei chemischen Substanzen üblich, umfasst die vorliegende Erfindung auch Derivate und Salze der erfindungsgemäß bereitgestellten Substanzen. Beispielsweise kann das gereinigte Mundsekret ein oder mehrere Additionen, Substitutionen und / oder Deletionen von Aminosäuren umfassen, wobei natürlich sichergestellt sein muss, dass die erfindungsgemäße medizinische Wirkung erhalten bleibt.
Die Gewinnung des beschriebenen Wirkstoffs erfolgt auch durch in der chemischen Verfahrenstechnik übliche Methoden. Hierzu gehören insbesondere Fraktionierungsverfahren; es sind aber auch andere Verfahren einsetzbar, beispielsweise immunologische Verfahren, um die gewünschten Substanzen aus dem. Mundsekret, herauszuholen
Bei dem erfindungsgemäßen Verfahren ist bevorzugt, dass das Mundsekret, vor der Fraktionierung homogenisiert wird, und es ist weiterhin bevorzugt, dass die Fraktionen vor der Weiterverarbeitung gefriergetrocknet bzw. tiefgekühlt und weiter bevorzugt lyophilisiert werden.
Die Wirkweise vom Mundsekret bzw. einzelner daraus säulenchromatographisch abgetrennter und / oder über das Molekulargewicht charakterisierter Substanzen kann durch Austestung dieser in entsprechenden gesunden und kranken humanen Zell - Linien erfolgen.
Gemäß der vorliegenden Erfindung stammen die verwendeten Substanzen bevorzugt aus dem gleichen Organismus wie die hierzu antagonistisch oder synergistisch wirkenden Substanzen und / oder wahlweise enthaltenen weiteren Wirksubstanzen. Auf diese Weise kann das effektive, von der Natur entwickelte Zusammenspiel oder Gegenspiel dieser Substanzen ausgenutzt werden.

Die Herstellung der erfindungsgemäßen pharmazeutischen Wirkstoffe kann so erfolgen , dass zunächst ein Mundsekret - Rohgemisch durch an sich bekannte Verfahren gewonnen wird und eine Fraktionierung des Rohgemisches durch ebenfalls an sich bekannte Fraktionierungsverfahren zur Auftrennung von Proteinen vorgenommen wird.. Dies dient dem Zweck die Enzyme und die hierzu antagonistisch oder synergistisch wirkenden Substanzen in möglichst voneinander getrennter Form beziehungsweise in getrennten Fraktionen zu erhalten. Anschließend können zur Herstellung eines pharmazeutischen Wirkstoffs verschiedene Fraktionen kombiniert werden oder einzelne Fraktionen können mit aus anderen Organismen stammenden Substanzen oder hierzu antagonistisch oder synergistisch wirkenden Substanzen kombiniert werden. Zur Herstellung eines pharmazeutischen Wirkstoffs können auch einzelne Fraktionen verwendet werden. Bevorzugt können als antagonistisch wirkende Substanzen Hyaluronidasen aus Schlangengiften, beispielsweise aus Kobragiften, eingesetzt werden. Dies kann kombiniert werden mit einer oder mehreren Fraktionen aus Substanzen die gewonnen wurden aus Echsen der unter a) bis e) genannten Arten.

Es ist auch möglich, zur Herstellung erfindungsgemäßer pharmazeutischer Wirkstoffe, die Fraktionen zusätzlich mit weiteren geeigneten Wirkstoffen und / oder mit in der Pharmazie üblichen Träger- und Hilfsstoffen zu kombinieren.

Zur Herstellung der erfindungsgemäßen pharmazeutischen Wirkstoffe können aus dem Tiersekret z.B. über säulenchromatographische Aufreinigung spezifische Komponenten (nekrotisch und zytotoxisch wirkende Substanzen), sowie natürliche hierzu antagonistisch wirkende Substanzen (Stoppsubstanzen), vom Phospholipase - und Hyaluronidase Typ selektiert werden.

Die erfindungsgemäßen Wirkstoffe sind antimikrobiell und stärken das Immunsystem.
Die erfindungsgemäßen Wirkstoffe sind nebenwirkungsarm, da sie mit dem menschlichen Körper und dessen Biochemie kompatibel sind.

Die Verteilung der einzelnen Wirkstoffe im Mundsekret der verschiedenen Tierarten ist etwa folgende:
a) ca. 60% Enzyme, Molekulargewicht etwa 18 kDa bis ca. 200 kDa
b) ca. 15 % Mucopolysaccharide
c) ca. 10% nicht bestimmbare organische Substanzen
d) ca. 5% Bakterien
e) ca. 10% Pilze
f) ca. 7% organische Substanzen wie Salze, Selen, Lithium, Seltenerden
g) ca. 2% Amine

Diese Verteilung ist bei allen genannten Arten ähnlich und vollkommen different zu der entsprechenden Verteilung anderer Arten und Gattungen.

Besonders vorteilhaft kann der Gehalt an aktiven Wirkstoffen im Mundsekret über die Ernährung der Tiere beeinflusst werden. Wenn die Nahrung der Tiere zu mindestens 10% Baumharze, wie beispielsweise Gummi arabicum, enthält, kann der Anteil an aktiven Wirkstoffen bedeutend erhöht werden. Neben einer erhöhten Wirkstoffkonzentration sind diese Harze auch für die Gesundheit der Tiere wichtig. Alternativ zu Gummi arabicum können auch Harze von Apfel- oder Birnbäumen verwendet werden. Auch Ahornsirup kann als Harz verwendet werden. Um eine Anreicherung von Pestiziden in den Mundsekreten zu vermeiden, sollten die Harze bevorzugt frei von Pestiziden sein, d.h. Bioqualität besitzen.

### 2.Bestandteil des erfindungsgemäßen Wirkstoffs

Der 2. Bestandteil des erfindungsgemäßen Wirkstoffes wird aus speziellen Weidenarten der Gattung Salix gewonnen, insbesondere
a) Salix ivigtutiana,
b) Salix reticulata, und
c) Salix pyrenaica.

Salix ivigtutiana wächst im Arktisch - Alpinen Garten Chemnitz. Sie gilt als kleinste Weide und wohl auch als kleinstes Gehölz der Welt.
1883 wurde in SW - Grönland in der Umgebung des kleinen Ortes Ivigtut von schwedischen Wissenschaftlern eine Weide gefunden, die mit einer Höhe von 10 bis 15 mm und Blättern von 5 bis 10 mm Länge und 2 bis 3 mm Breite wohl als kleinster Vertreter der Gattung Salix angesehen werden kann. Diese von LUNDSTRÖM als Salix ivigtutiana beschriebene Pflanze konnte nochmals 1924 von einer schwedischen Expedition gefunden und in einigen Exemplaren in den Botanischen Garten Kopenhagen gebracht werden. Sie ist seither in der Natur nicht wieder gesichtet worden und gilt als verschollen.
1978 erhielt Walter Meusel vom damaligen Leiter des Kopenhagener Botanischen Gartens, Herrn Olaf Olsen, einige Stecklinge dieser seltenen Art, deren Kultur in Chemnitz gelang. Diese Pflanze existiert inzwischen auch im Botanischen Garten Kopenhagen nicht mehr, konnte jedoch im Arktisch - Alpinen Garten Chemnitz vermehrt und bereits an mehrere Spezialisten weitergegeben werden. (Stand 1995 ). Somit ist der Fortbestand dieser sicher nicht zuletzt auch für die Wissenschaft interessanten Pflanze gesichert, und es wären gegebenenfalls sogar Reintegrierungsversuche am natürlichen Standort in Zusammenarbeit mit den grönländischen Naturschutzorganen denkbar.

Salix reticulata gehört zur Ordnung der Salicales (Weidenartige), Familie Salicaceae (Quellergewächse) und zur Gattung Salix (Weide).
Der deutsche Name für diese Pflanze heißt Netz - Weide.
Gemäß den Zeigerwerten nach Ellenberg handelt es sich bei dieser Art um eine Lichtpflanze, einen Basen- und Kalkanzeiger. Sie ist auf stickstoffarmen Standorten häufiger und nicht salzertragend. Sie ist ein Kälte - bis Kühlezeiger und Frische - bis Feuchtezeiger.
Ihrer Soziologie nach zählt sie zu den Arabidion caeruleae (Schneebodengesellschaften).
Die Höhe von Salix reticula beträgt 1 bis 10 cm. Es handelt sich um einen zwergigen kriechenden Strauch mit sich bewurzelnden (der Spross liegt auf dem Boden und bildet an vielen Stellen Wurzeln) Sprossen und rundlich - eiförmigen, 1 bis 4 cm langen, glänzenden, dunkelgrünen Blättern. Die Oberseite ist auffällig geadert, die Unterseite ist netzartig weißlich behaart. Im Frühling werden schlanke, 2,5 cm lange, gelbe, an der Spitze rosafarbene lang gestielte Kätzchen gebildet.Diese Pflanze bildet männliche und weibliche Blüten aus.
Das Vorkommen von Salix reticulata ist Skandinavien und Finnland, Nordasien und Nordamerika. Diese Pflanze , die oberhalb der Baumgrenze wächst, liebt Schneekuhlen mit ihren feuchten Böden, ebenso feuchte Felshänge und Geröllhalden.
Die netzartige behaarte Blattunterseite ist der perfekte Klimaschutz für Salix reticulata. Sie vermindert die Windgeschwindigkeit an der Blattoberfläche und beugt damit Austrocknung vor.
Salix Pyrenaica besiedelt feuchte Orte wie zum Beispiel Rasen und Torfmoore. Diese Pflanze wächst am Boden ausgebreitet unter Sträuchern, sehr verzweigt und gewunden. Die behaarten jüngeren Zweig sind braun-rot, die Knospen sind unbehaart. Die Blüten sind grünlich und behaart. Salix pyrenaica trägt Früchte in der Form von konischen behaarten gräulichen Kapseln. Sie kommt nur in den Pyrenäen vor.

Von den genannten Pflanzen wird nur die frische lebende Rinde verwendet. Diese wird bevorzugt mit einem scharfen Damastmesser, also im Wesentlichen einem Messer mit einer dünnen Klinge aus Damaszener ― Stahl, gewonnen. Besonders geeignet hierfür ist japanischer Damast. Die Klinge sollte möglichst scharf sein, damit der Zellverband effektiver durchtrennt werden kann. Somit kann die Entnahme der Rinde verletzungsarm bzw. verletzungsfrei vorgenommen werden. Neben Vorteilen für die weitere Verarbeitung der entnommenen Rinde kann so sichergestellt werden, dass der Baum nicht verletzt wird und regelmäßig geerntet werden kann.
Bevorzugt ist weiter, dass die Rinde in den Abendstunden vorsichtig in schmalen Streifen aus der Rinde der betreffenden Pflanzen geschnitten werden, wobei der unterste Schnitt ein Stück über dem Boden liegen muss und die Breite des abgeschälten Rindenstreifens etwa zwei Drittel des Durchmessers des Stammes nicht überschreiten darf.
Die Gewinnung der Rinde in den Abendstunden ist deshalb vorteilhaft, da die Pflanzen zu dieser Zeit einen anaeroben Stoffwechsel haben.
Durch diese Maßnahmen ist gewährleistet, dass die jeweilige Pflanze diese Prozedur überlebt.
Sind auf diese Weise ca. 5 Gramm Rinde gesammelt wird diese pulverisiert und homogenisiert.

### 3.Bestandteil des erfindungsgemäßen Wirkstoffs

Der 3. Bestandteil des erfindungsgemäßen Wirkstoffes wird aus dem Mundsekret von einer der folgenden Eidechsen-Gattungen (Lacertidae) gewonnen.
a) Podarcis
b) Lacerta

Von den Eidechsen-Gattungen werden lediglich die der Gattung Podarcis und Lacerta verwendet. Besonders bevorzugt werden dabei aus der Gattung Podarcis die Arten Podarcis muralis, Podarcis tiliguerta, Podarcis lilfordi und Podarcis peloponnesiaca. Von der Gattung Lacerta werden die Arten Lacerta viridis, Lacerta bilineata, Lacerta trilineata und Lacerta agilis bevorzugt verwendet.

Im folgenden soll auf einige der oben genannten Arten näher eingegangen werden, ohne jedoch auf diese Arten beschränkt zu sein.

Die Mauereidechse Podarcis muralis gehört zur Familie der Echten Eidechsen.
Sie besitzt einen schwarzen Kopf, einen schlanken, mäßig abgeflachten Körper und einen langen Schwanz. Die Kopfrumpflänge beträgt bis 7,5cm , die Schwanzlänge beträgt das 1,5 bis 2,3 - fache dieser Länge.
Die Färbung ist sehr variabel und besteht auf der Oberseite aus den unterschiedlichsten Brauntönen. Die Männchen zeigen auch manchmal eine grünliche Zeichnung und am Übergang zwischen Flanken und Bauch auch einzelne blaue Schuppen. Vorherrschend sind an den Flanken dunkle Töne die zum Rücken meist mit einer dunklen Längslinie abschließen. Der Rücken ist meist heller und weist manchmal in der Mitte eine durchbrochene Längslinie auf. Bei den Männchen lösen sich diese Längszeichnungsmuster oft auch in Netzzeichnungsmuster auf.
Die Bauchfärbung reicht bei den Weibchen von creme -weiß bis kupferbraun und ist fast einfarbig. Bei den Männchen kann der Bauch auch leuchtend gelb bis orange gefärbt sein und ist oft stark dunkel gepunktet bis fast ganz schwarz.
Die Mauereidechse kommt von Nordostspanien bis Mittelspanien ostwärts über Mitteleuropa und Südeuropa vor und in den Balkanländern bis zur Westküste des Schwarzen Meeres. Im Norden reicht ihr Vorkommen bis Südengland.

Der erfindungsgemäße Wirkstoff wurde aus Populationen von Podarcis muralis gewonnen, die aus folgenden Gebieten stammen:
Dem Gebiet um den Garda - See (Norditalien), dem Tenno - See (kleiner See oberhalb von Riva del Garda),und dem Ledro - See (gelegen im Hinterland des Bereichs Garda - See). Ferner wurde mit Populationen aus dem Gebiet um Bozen und aus Istrien gearbeitet.

Die Mauereidechse besiedelt warme Biotope an Fels - und Weinhängen, Bahn - und Straßenböschungen und Mauern bis in den Bereich von Siedlungen und Städten, aber auch wärmebegünstigte Wälder.
Die Mauereidechse ist unter den einheimischen Eidechsenarten die agilste und behändetste. Sie ernährt sich von Insekten, Spinnen und anderen Gliedertieren, geht aber auch an süße Früchte.

Etwa vier Wochen nach der Winterruhe beginnt im März die Paarungszeit. Die Männchen verteidigen dabei Reviere. Die Weibchen legen in selbst gegrabene Löcher und Mauerritzen 2 bis 10 Eier. Je nach der Verbreitung sind 2 bis 3 Gelege möglich. Die Winterruhe beginnt je nach Witterung meist im Oktober. Manchmal sonnen sich einzelne Tiere an warmen Tagen auch im Winter und Vorfrühling.

Die Smaragdeidechse (Lacerta viridis) zählt zu den größten Exemplaren unter den Eidechsen (Familie Lacertidae). Mit einer Körperlänge von bis zu fünfzig Zentimetern, wovon allerdings etwa zwei Drittel auf den Schwanz entfallen, wird sie nur von drei der über zweihundert verschiedenen Eidechsenarten an Größe noch übertroffen.
Die eigentliche Heimat des leuchtendgrünen Reptils , dessen Männchen zur Paarungszeit ein prächtig blaues Haupt zur Schau tragen, sind die im Norden ans Mittelmeer angrenzenden Länder zwischen Portugal im Westen und Israel im Osten. Nördlich der Alpen, in Mitteleuropa ist diese Art nur ganz lückenhaft vertreten.

Der erfindungsgemäße Wirkstoff wurde im Fall der Lacerta viridis aus Populationen gewonnen, die im Bereich des norditalienischen Gardasees und hier speziell aus dem Bereich des Tenno -Sees, eines kleinen Bergsees oberhalb von Riva del Garda, stammen.

Innerhalb ihres weiten Verbreitungsgebiets bewohnt die wärmeliebende Smaragdeidechse gut besonntes, trockenes Gelände, buschbestandene Wiesen, Wegborde, Geröllhalden, lichte Hecken oder Felshänge. Hier jagt das flinke Tier am Tag nach Insekten, Spinnen Würmern und Schnecken. Hier und da packt es auch kleinere Eidechsen. In besonders gefährlichen Situationen verliert die Smaragdeidechse ihren Schwanz. Wie alle Eidechsen vermag sie den Verlust des Schwanzes aktiv herbeizuführen, wenn sie von einem Angreifer unmittelbar bedroht wird. Als solche kommen, neben Marder und Wiesel, vor allem Greifvögel in Frage. Vom sechsten Wirbel an weist jeder Schwanzwirbel der Smaragdeidechse eine vorgebildete Bruchstelle auf. Durch ein kräftiges, abruptes Zusammenziehen der Ringmuskulatur der einen oder anderen Schwanzpartie kann sie darum je nach Bedarf einen kleineren oder größeren Teil des Schwanzes abwerfen. Das autonome Nervensystem des Schwanzfragments lässt den abgetrennten Körperteil noch bis zu zwanzig Minuten lang heftig zappeln. Während dieser die Aufmerksamkeit des Verfolgers auf sich zieht, kann das nunmehr schwanzlose Reptil die Flucht ergreifen.. Der behänden Eidechse wächst nach einem solchen Vorfall bald ein neuer Schwanz. Anstelle der ehemaligen Wirbelsäule wird allerdings ein ungegliederter Knorpelstab als Stützelement aufgebaut, von dem fortan kein Teil mehr abgetrennt werden kann.

Der erfindungsgemäße Wirkstoff wird bei den angeführten Eidechsenarten auf die folgende Weise gewonnen, wobei die Anweisung für Rechtshänder gilt:
a) Die jeweilige Eidechse bei einer Umgebungstemperatur von 22 bis 26 ° C in die linke Hand nehmen.
b) Der Eidechse ein steriles Wattestäbchen nähern und warten bis das Tier in dieses Wattestäbchen beißt.
c) Die Eidechse zurück in das verwendete Behältnis setzen.
d) Das betreffende Wattestäbchen für ca. 2 Stunden zur Analyse in etwa in etwa 1 ml Reinstwasser legen.
e) Das Wasser unter sterilen Bedingungen verdunsten lassen.
f) Den erhaltenen Wirkstoff in 15 ml einer isotonischen NaCl-Lösung geben.
g) Diese Lösung bei 121°C autoklavieren
h) Nach dem Autoklavieren 10 ml Tarantula D4 (als Zelltransmitter) zusetzen.
Die isotonische NaCl-Lösung enthält NaCl in einer isotonischen Konzentration, d.h. sie enthält NaCl zu 0,9%.
Das Melken der jeweiligen Eidechse soll hierbei nicht öfter als einmal in einem Zeitraum von 4 Wochen erfolgen.
Nach 4 Monaten ist bei dem genannten Zeitabstand eine Pause von 3 Monaten einzuhalten.

Wie bereits für die Geckos erwähnt, kann der Gehalt an aktiven Wirkstoffen im Mundsekret vorteilhaft über die Ernährung der Tiere beeinflusst werden. Auch hier kann der Anteil an aktiven Wirkstoffen bedeutend erhöht werden, wenn die Nahrung der Tiere Baumharze, wie beispielsweise Gummi arabicum, enthält. Alternativ zu Gummi arabicum können, wie bereits oben erwähnt, auch Harze von Apfel- oder Birnbäumen verwendet werden. Auch Ahornsirup kann als Harz verwendet werden.

Um eine Anreicherung von Pestiziden in den Mundsekreten zu vermeiden, sollten die Harze bevorzugt frei von Pestiziden sein, d.h. Bioqualität besitzen.
Positiv auf die Wirkstoffkonzentration bei den Eidechsen kann sich auch der Zusatz von Ameisen-Geschlechtstieren und -Puppen auswirken. Dadurch wird die Wirkstoffkonzentration erhöht. Alternativ können auch giftstachellose Drohnen der Honigbiene als Nahrungsergänzung zur Erhöhung der Wirkstoffkonzentration verfüttert werden.

Eine besonders bevorzugte Mischung als Nahrung für Eidechsen ist ein Frucht-Babybrei, dem bis zu 5% einer gesättigten Lösung aus Gummi arabicum beigemischt wird.

Bei Tarantula D4 handelt es sich um eine homöopathische Substanz. Bekanntermaßen erhält in der Homöopathie der Kranke das betreffende Medikament nicht in seiner Urtinktur, sondern in einer Verdünnungsstufe. Der Begründer der Homöopathie, Samuel Christian Hahnemann, machte die paradox anmutende Beobachtung, dass sich die Wirkung einer Arznei umgekehrt proportional zur Konzentration verhält. Je stärker die Stammlösung verdünnt wird, desto wirksamer wird sie.
Die D - Verdünnungsstufen werden hergestellt, indem 1/10 der Stammlösung mit 9/10 Alkohol aufgefüllt und dann geschüttelt wird. Dadurch erhält man die erste Verdünnung D1. Von dieser Verdünnung nimmt man erneut 1/10 und verschüttelt es mit 9 Teilen Alkohol, und man erhält eine D2.- Verdünnung. So wird diese Prozedur fortgesetzt bis schließlich hohe Potenzen wie z.B. D 200 entstehen. Dennoch handelt es sich um hochwirksame Arzneien. Jedoch um hierfür nach einem Erklärungsmodell zu suchen muss man sich von der Physik, die mit einfachen linearen stoffbezogenen Thesen arbeitet, verabschieden.

Wahlweise kann der erfindungsgemäße Wirkstoff eine zu dem jeweiligen Mundsekret antagonistische bzw. synergistische und / oder Durchdringungssubstanz aus dem. Mundsekret, der betreffenden Tierart enthalten.
Die antagonistisch bzw. synergistisch wirkende Substanz ist bevorzugt eine Phospholipase oder eine Hyaluronidase oder eine Kombination beider Substanzen.

Weiterhin ist möglich, dass die antagonistisch bzw. synergistisch wirkende Substanz eine Mischung aus den, in anderen Arten, vorhandenen Phospholipasen und Hyaluronidasen und / oder Toxinen ist.
Es kann das Mundsekret und die hierzu antagonistisch und / oder synergistisch wirksame Substanz durch ein Fraktionierungsverfahren aus dem Gesamt- Cocktail des Mundraumsekrets erhalten werden, und es ist weiterhin möglich, dass der pharmazeutische Wirkstoff ein Mundsekret und eine hierzu antagonistisch oder synergistisch wirkende Substanz enthält, die aus verschiedenen Fraktionen stammen. Dadurch kann der pharmazeutische Wirkstoff in seiner Wirkung vorteilhafterweise auf die zu behandelnde MS - Art abgestimmt werden.
Das Mundsekret und die hierzu antagonistisch und / oder synergistisch wirkende Substanz können durch an sich bekannte Fraktionierungsverfahren zur Auftrennung von Proteinen aus dem Mundssekret, erhalten werden. Es ist möglich, dass die gewonnen Substanzen und die hierzu antagonistisch oder synergistisch wirkende Substanz durch Gelchromatographie, HPC, Affinitätschromatographie und / oder lonenaustauschchromatographie erhalten werden.

Es ist weiter bevorzugt, dass der erfindungsgemäße pharmazeutische Wirkstoff übliche Träger - und Hilfsstoffe enthält, wie Antibiotika, Antimykotika, Antituberkulotika, Mittel gegen Parasiten, Zytostatika, Aminosäuren, die Wundheilung begünstigende Enzyme und / oder Mitosehemmstoffe. Bevorzugt sind hierbei Penicillin/Streptomycin, Polymyxin / Gentalmycin (5%), Mitopodozid, Vinca rosea - Alkaloide, Bromelaina oder Bromelains.
In dem erfindungsgemäßen pharmazeutischen Wirkstoff werden das Mundsekret, und die antagonistisch oder synergistisch wirkende Substanz in Kombination mit der homöopathischen Substanz miteinander eingesetzt. Es ist aber auch möglich, die Einzelsubstanzen in pharmazeutischen Wirkstoffen zu benutzen und sich hierbei die speziellen Wirkungen der Einzelsubstanzen für eine therapeutische Anwendung nutzbar zu machen.
Es ist auch möglich die beschriebenen Wirkstoffe chemisch - synthetisch oder durch gentechnologische Methoden in rekombinierter Form herzustellen. Wie bei chemischen Substanzen üblich, umfasst die vorliegende Erfindung auch Derivate und Salze der erfindungsgemäß bereitgestellten Substanzen. Beispielsweise kann das gereinigte Mundsekret ein oder mehrere Additionen, Substitutionen und / oder Deletionen von Aminosäuren umfassen, wobei natürlich sichergestellt sein muss, dass die erfindungsgemäße medizinische Wirkung erhalten bleibt.
Die Gewinnung des beschriebenen Wirkstoffs erfolgt auch durch in der chemischen Verfahrenstechnik übliche Methoden. Hierzu gehören insbesondere Fraktionierungsverfahren; es sind aber auch andere Verfahren einsetzbar, beispielsweise immunologische Verfahren, um die gewünschten Substanzen aus dem. Mundsekret, herauszuholen

Bei dem erfindungsgemäßen Verfahren ist bevorzugt, dass das Mundsekret, vor der Fraktionierung homogenisiert wird, und es ist weiterhin bevorzugt, dass die Fraktionen vor der Weiterverarbeitung gefriergetrocknet bzw. tiefgekühlt und weiter bevorzugt lyophilisiert werden.
Um ungewollte Zellzerstörungen zu verhindern, kann erfindungsgemäß in Abhängigkeit von Art und Größe des zu behandelnden Zellbereichs ein Abgleich bezüglich absoluter und relativer Mengen der Bestandteile erfindungsgemäßen Wirkstoffes in vitro an lebenden menschlichen Zellen (gesund und krank) des zu therapierenden Gewebetyps erfolgen. Hierbei kommt der Beachtung der Ausbreitungstendenz die größte Bedeutung zu. Diese kann im Vergleich der Gewebsfestigkeit zu dem, den Krankheitsherd umgebenden, Gewebe in Vorversuchen abgeklärt werden.
Die Wirkweise vom Mundsekret bzw. einzelner daraus säulenchromatographisch abgetrennter und / oder über das Molekulargewicht charakterisierter Substanzen kann durch Austestung dieser in entsprechenden gesunden und kranken humanen Zell - Linien erfolgen.

Gemäß der vorliegenden Erfindung stammen die verwendeten Substanzen bevorzugt aus dem gleichen Organismus wie die hierzu antagonistisch oder synergistisch wirkenden Substanzen und / oder wahlweise enthaltenen weiteren Wirksubstanzen. Auf diese Weise kann das effektive, von der Natur entwickelte Zusammenspiel oder Gegenspiel dieser Substanzen ausgenutzt werden.

Die Herstellung der erfindungsgemäßen pharmazeutischen Wirkstoffe kann so erfolgen , dass zunächst ein Mundsekret ― Rohgemisch durch an sich bekannte Verfahren gewonnen wird und eine Fraktionierung des Rohgemisches durch ebenfalls an sich bekannte Fraktionierungsverfahren zur Auftrennung von Proteinen vorgenommen wird.. Dies dient dem Zweck die Enzyme und die hierzu antagonistisch oder synergistisch wirkenden Substanzen in möglichst voneinander getrennter Form beziehungsweise in getrennten Fraktionen zu erhalten. Anschließend können zur Herstellung eines pharmazeutischen Wirkstoffs verschiedene Fraktionen kombiniert werden oder einzelne Fraktionen können mit aus anderen Organismen stammenden Substanzen oder hierzu antagonistisch oder synergistisch wirkenden Substanzen kombiniert werden. Zur Herstellung eines pharmazeutischen Wirkstoffs können auch einzelne Fraktionen verwendet werden. Bevorzugt können als antagonistisch wirkende Substanzen Hyaluronidasen aus Schlangengiften, beispielsweise aus Kobragiften, eingesetzt werden. Dies kann kombiniert werden mit einer oder mehreren Fraktionen aus Substanzen die gewonnen wurden aus Eidechsen der unter a) bzw. b) genannten Gattungen.

Es ist auch möglich, zur Herstellung erfindungsgemäßer pharmazeutischer Wirkstoffe, die Fraktionen zusätzlich mit weiteren geeigneten Wirkstoffen und / oder mit in der Pharmazie üblichen Träger- und Hilfsstoffen zu kombinieren.

Zur Herstellung der erfindungsgemäßen pharmazeutischen Wirkstoffe können aus dem Tiersekret z.B. über säulenchromatographische Aufreinigung spezifische Komponenten (nekrotisch und zytotoxisch wirkende Substanzen), sowie natürliche hierzu antagonistisch wirkende Substanzen (Stoppsubstanzen), vom Phospholipase - und Hyaluronidase Typ selektiert werden.

Die erfindungsgemäßen Wirkstoffe sind antimikrobiell und stärken das Immunsystem.
Die erfindungsgemäßen Wirkstoffe sind nebenwirkungsarm, da sie mit dem menschlichen Körper und dessen Biochemie kompatibel sind.

### Herstellung des erfindungsgemäßen Wirkstoffs

a) Es wird mit der Herstellung einer Stammlösung begonnen die aus jeweils zu einem Drittel aus der pulverisierten und homogenisierten Rinde von Pflanzen der Art Salix ivigtutiana, der Art Salix reticulata und der Art Salix pyrenaica besteht. Die Gewinnung ist in der Beschreibung des 2.Bestandteils erläutert.
b) Diese Lösung wird in steriler Umgebung unter leichtem Rühren auf 50°C erwärmt.
c) Hierauf wird eine gesättigte Lösung aus dem Mundsekret von Geckos einer der in der Beschreibung des 1. Bestandteils genannten Arten bereitgestellt.
d) Im Folgenden werden von jeder der drei unter a) beschriebenen Lösungen jeweils 5 mL bei 50°C langsam in die Lösung nach b) eingerührt.
e) Die so erhaltene Lösung muss 50 Stunden bei Raumtemperatur ruhen, damit sich die Proteine in der Lösung entfalten. Danach muss eine Abkühlung auf 10°C bis 15°C erfolgen.
f) Hierauf muss von den in der Beschreibung des 3. Bestandteils genannten beiden Eidechsenarten zu gleichen Teilen, wie dort beschrieben, eine Stammlösung hergestellt werden.
g) Hierauf sind 5 mL dieser Stammlösung unter Luftabschluss (Stickstoff - Umgebung) in die unter e) beschriebene Lösung zu rühren.

Der derart hergestellte Wirkstoff kann im Kühlschrank bei zwischen 0 und 7°C bei voller Wirksamkeit über vier Monate gelagert werden.

## Patentansprüche

1. Pharmazeutischer Wirkstoff zur Behandlung von Multipler Sklerose, enthaltend in einer pharmazeutisch wirksamen Menge
a) die Rinde von Pflanzen der Gattung Salix,
b) Mundsekret von Geckos der Gattung Rhacodactylus und
c) Mundsekret von Tieren der Gattungen Podarcis und Lacerta.

2. Pharmazeutischer Wirkstoff nach Anspruch 1,
**dadurch gekennzeichnet,**
**dass** die Rinde von Pflanzen der Arten Salix ivigtutiana und/oder Salix reticulata und/oder Salix pyrenaica stammt.

3. Pharmazeutischer Wirkstoff nach Anspruch 1 oder 2,
**dadurch gekennzeichnet,**
**dass** das Mundsekret von Geckos der Arten Rhacodactylus auriculatus und/oder Rhacodactylus leachianus und/oder Rhacodactylus henkeli und/oder Rhacodactylus chahoua und/oder Rhacodactylus ciliatus verwendet wird.

4. Pharmazeutischer Wirkstoff nach einem der Ansprüche 1 bis 3,
**dadurch gekennzeichnet,**
**dass** die Tiere der Gattung Podarcis ausgewählt sind aus der Gruppe, bestehend aus Podarcis muralis, Podarcis tiliguerta, Podarcis lilfordi und Podarcis peloponnesiaca.

5. Pharmazeutischer Wirkstoff nach einem der Ansprüche 1 bis 3,
**dadurch gekennzeichnet,**
**dass** die Tiere der Gattung Lacerta ausgewählt sind aus der Gruppe, bestehend aus Lacerta viridis, Lacerta bilineata, Lacerta trilineata und Lacerta agilis.

6. Pharmazeutischer Wirkstoff nach Anspruch 4,
**dadurch gekennzeichnet,**
**dass** Tiere der Art Podarcis muralis verwendet werden, und diese
a) aus dem Bereich des oberitalienischen Gardasees, oder
b) aus dem Bereich des oberitalienischen Tennosees, oder
c) aus dem Bereich des oberitalienischen Ledrosees, oder
d) aus dem Bereich der oberitalienischen Stadt Bozen, oder
e) aus dem Gebiet von Istrien,
stammen.

7. Pharmazeutischer Wirkstoff nach Anspruch 5,
**dadurch gekennzeichnet,**
**dass** Tiere der Art Lacerta viridis verwendet werden, und diese
a) aus dem Bereich des oberitalienischen Gardasees oder
b) aus dem Bereich des oberitalienischen Tennosees
stammen.

8. Pharmazeutischer Wirkstoff nach einem oder mehreren der vorhergehenden Ansprüche,
**dadurch gekennzeichnet,**
**dass** dem Mundsekret der Tiere der Gattung Podarcis oder Lacerta in einer pharmazeutisch wirksamen Menge Tarantula D4 zugesetzt wird.

9. Verfahren zur Herstellung eines pharmazeutischen Wirkstoffs zur Behandlung von Multipler Sklerose gemäß einem oder mehreren der vorhergehenden Ansprüche,
**dadurch gekennzeichnet, dass**
1) eine Stammlösung hergestellt wird, die jeweils zu einem Drittel aus der pulverisierter und homogenisierter Rinde von Pflanzen der Art Salix ivigtutiana, der Art Salix reticulata und der Art Salix pyrenaica besteht, und dass
2) diese Lösung in steriler Umgebung unter leichtem Rühren auf 50°C erwärmt wird, und dass
3) eine gesättigte Lösung aus dem Mundsekret von Geckos der folgenden Arten bereitgestellt wird:
a) Rhacodactylus auriculatus (Höckerkopfgecko)
b) Rhacodactylus leachianus (Riesengecko)
c) Rhacodactylus leachianus henkeli (Henkels Riesengecko)
d) Rhacodactylus chahoua (Flechtengecko) und dass
4) in die Lösung unter 3) jeweils 5 mL der unter 1) beschriebenen Lösungen bei 50°C langsam eingerührt werden. und dass
5) die so erhaltene Lösung 50 Stunden bei Raumtemperatur ruht und danach auf eine Temperatur von 10°C bis 15°C abgekühlt wird, und dass
6) von dem Mundsekret von Echsen der Gattungen Podarcis und Lacerta zu jeweils gleichen Teilen eine Stammlösung hergestellt wird, und dass
7) 5 mL der Lösung unter 6) unter Luftabschluss in die unter 5) beschriebene Lösung gerührt wird.

10. Verfahren zur Herstellung eines pharmazeutischen Wirkstoffs zur Behandlung von Multipler Sklerose nach Anspruch 9,
**dadurch gekennzeichnet, dass**
die Gewinnung der Rinde zur Herstellung der Stammlösung unter 1) aus dünnen Streifen der Rinde junger Äste erfolgt, wobei die Breite der Rindenstreifen unterhalb von zwei Dritteln des jeweiligen Astdurchmessers bleibt, der unterste Rand des Rindenstreifens in geringer Entfernung vom Erdboden liegt und die Rinde in den Abendstunden mit einem Damastmesser abgeschält wird.

11. Verfahren zur Herstellung eines pharmazeutischen Wirkstoffs zur Behandlung von Multipler Sklerose nach Anspruch 9 oder 10,
**dadurch gekennzeichnet, dass**
die Gewinnung des Mundsekrets zur Herstellung der Lösung unter 3) **dadurch** erfolgt, dass von dem jeweiligen Gecko ein Wattestäbchen abgeleckt wird und dieses in einer isotonischen NaCl-Lösung, einer Mischung, enthaltend eine isotonische NaCl-Lösung und Tarantula D4, oder sterilem Wasser ausgeschwenkt wird.

12. Verfahren zur Herstellung eines pharmazeutischen Wirkstoffs zur Behandlung von Multipler Sklerose nach einem oder mehreren der Ansprüche 9-11,
**dadurch gekennzeichnet, dass**
die Gewinnung des Mundsekrets zur Herstellung der Stammlösung unter 6) erfolgt durch die folgenden Verfahrensschritte:
a) der jeweiligen Eidechse erlauben, bei einer Umgebungstemperatur von 22 bis 26°C, in ein steriles Wattestäbchen zu beißen;
b) das betreffende Wattestäbchen für ca. 2 Stunden zur Analyse in etwa 1 ml Reinstwasser legen;
c) das Wasser unter sterilen Bedingungen verdunsten lassen;
d) den erhaltenen Wirkstoff in 15 ml einer isotonischen NaCl-Lösung geben;
e) diese Lösung bei 121 °C autoklavieren; und
f) nach dem Autoklavieren 10 ml Tarantula D4 (als Zelltransmitter) zusetzen.
